# EUROPÄISCHE PATENTSCHRIFT

(11) **EP 0 962 454 B1**
(45) Veröffentlichungstag und Bekanntmachung des Hinweises auf die Patenterteilung: **09.10.2002**
(21) Anmeldenummer: 99109889.8
(22) Anmeldetag: 20.05.1999
(51) Int. Cl.: C07D 273/04, C07D 251/34, C08G 18/02

(54) **Verfahren zur Herstellung Iminooxadiazindiongruppen enhaltender Polyisocyanate**
Process for the preparation of imino-oxadiazine-dione groups containing polyisocyanates
Procédé pour la préparation de polyisocyanates contenant des groupes imino-oxadiazine-dione

(30) Priorität: 02.06.1998 DE 19824490; 02.06.1998 DE 19824485
(43) Veröffentlichungstag der Anmeldung: 08.12.1999
(73) Patentinhaber: Bayer Aktiengesellschaft, 51368 Leverkusen (DE)
(72) Erfinder: Richter, Frank, Dr., 51373 Leverkusen (DE); Groth, Stefan, Dr., 51373 Leverkusen (DE); Stelter, Eberhard, Dr., 50735 Köln (DE); Litz, Wilfried, Dr., 51107 Köln (DE)

(56) Entgegenhaltungen:
- EP-A- 0 339 396
- EP-A- 0 355 479
- EP-A- 0 379 914
- EP-A- 0 447 074
- EP-A- 0 798 299
- EP-A- 0 841 088
- EP-A- 0 896 009
- NAMBU Y ET AL: "Synthesis of novel aromatic isocyanurates by the fluoride-catalyzed selective trimerization of isocyanates" JOURNAL OF ORGANIC CHEMISTRY, Bd. 58, Nr. 7, 1993, Seiten 1932-4, XP002113530

## Beschreibung

Die Erfindung betrifftein Verfahren zur Herstellung Iminooxadiazindiongruppen enthaltender Polyisocyanate.

Iminooxadiazindiongruppen enthaltende Polyisocyanate (asymmetrische Trimere) sind hochwertige Rohstoffe u.a. für die Herstellung von Polyurethan-Lacken und Beschichtungen (DE-A 19 611 849). Sie sind, allerdings nur als Nebenkomponente, ein ständiger Begleiter der lange bekannten Isocyanurate (symmetrischen Trimeren).

Isocyanat-Oligomere mit signifikant erhöhtem Iminooxadiazindiongehalt sind Gegenstand der älteren Anmeldung (DE-A 19 611 849) in der auch ihre vorteilhaften Eigenschaften, z.B. als Rohstoff zur Herstellung von Polyurethan-Lacken und Beschichtungen, ausführlich beschrieben werden. So sind Iminooxadiazindiongruppen enthaltende Polyisocyanate die am niedrigsten viskosen, mindestens NCO-trifunktionellen (Di)isocyanat-Oligomeren.

In der DE-A 19 611 849 werden ganz allgemein Hydrogen(poly)fluoride der allgemeinen Formel {M[nF⁻·(HF)ₘ]}, wobei ^{m}/ₙ> 0 ist und M ein n-fach geladenes Kation bzw. einen n-wertigen Rest darstellt, als Katalysator zur Isocyanattrimerisierung unter bevorzugter Iminooxadiazindiongruppenbildung vorgeschlagen.

Diese Verfahrensweise ist jedoch mit dem Nachteil behaftet, daß zur Herstellung der Katalysatoren, sie erfolgt in der Regel aus den entsprechenden Fluoriden {M[nF⁻]}, wobei M ein n-fach geladenes Kation darstellt, HF gehandhabt werden muß. Letzterer Umstand limitiert die technische Durchführbarkeit des gesamten Verfahrens, da zur Handhabung von, gegebenenfalls wasserfreier, Fluorwasserstoffsäure einerseits besondere Schutzmaßnahmen erforderlich sind, die die Herstellung des Katalysators sehr aufwendig machen und andererseits, aufgrund der Korrosivität des Materials, bestimmte Anforderungen an die Auswahl der Reaktoren resultieren, in denen der Katalysator hergestellt und eingesetzt werden kann.

Diese Umstände stehen einer breiten, gefahrlosen Durchführung des Verfahrens der Isocyanattrimerisierung unter anteiliger Iminooxadiazindiongruppenbildung entgegen.

Aufgabe der Erfindung war es daher, ein Verfahren zur Verfügung zu stellen, das zum einen dadurch gekennzeichnet ist, daß zur Herstellung der Katalysatoren keine Fluorwasserstoffsäure gehandhabt werden muß aber andererseits trotzdem Produkte mit einem hohen Gehalt an Iminooxadiazindiongruppen im Trimerengemisch resultieren (der Terminus "Trimerengemisch" bezieht sich in der vorliegenden Schrift immer auf die Summe aus Isocyanurat und Iminooxadiazindion). Ein hoher Iminooxadiazindiongruppengehalt im Sinne der vorliegenden Erfindung bezeichnet Produkte mit mindestens 30 % an Iminooxadiazindiongruppen im Trimerengemisch.

Diese Aufgabe konnte durch das nachfolgend näher beschriebene Verfahren, der Katalyse der Isocyanat-Trimerisierung durch quaternäre Ammonium- bzw. Phosphoniumfluoride, gelöst werden.

Gegenstand der Erfindung ist ein Verfahren zur Herstellung von Polyisocyanaten die mindestens 30 mol-% an Iminooxadiazindiongruppen (asymmetrische Trimere) im Trimerengemisch enthalten durch katalytisch induzierte Trimerisierung organischer Di- oder Polyisocyanate mit einem (mittleren) Molekulargewicht von 140 - 600 g/mol mit unabhängig voneinander aliphatisch, cycloaliphatisch und/oder araliphatisch gebundenen Isocyanatgruppen, dadurch gekennzeichnet, daß als Trimerisierungskatalysator quaternäre Ammonium- sowie Phosphoniumfluoride der Formel

R₄E⁺ F⁻ (I),

wobei
- E: für N oder P steht und
- R: für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Stickstoff- bzw. Phosphoratom auch gesättigte oder ungesättigte Cyclen bilden können,
in Abmischung mit Solvatisierungsmitteln S für das Fluoridanion eingesetzt werden, wobei S eine reine Verbindung oder ein Gemisch verschiedener Stoffe aus der Gruppe der protischen Verbindungen mit einem pKₛ-Wert größer als 2 (bestimmt in H₂O bei 25°C) sowie Oxalsäure, jedoch kein höherfunktioneller Alkohol (Di- bzw. Polyol) sowie HF, sein kann, unter der Voraussetzung, daß das molare Verhältnis von S zu Fluoridion, F⁻, den Wert 10 nicht überschreitet.

Vorzugsweise werden im erfindungsgemäßen Verfahren als zu trimerisierende Isocyanatkomponente aliphatische Diisocyanate eines Molekulargewichts(bereichs) von 140 - 300 g/mol als reine Verbindungen oder in beliebiger Abmischung untereinander eingesetzt.

Die Verfahrensprodukte weisen vorzugsweise einen Gehalt von mindestens 35 %, besonders bevorzugt von mindestens 40 mol-% an Iminooxadiazindiongruppen (asymmetrische Trimere) im Trimerengemisch auf.

Besonders bevorzugt werden als Trimerisierungskatalysator quaternäre Ammonium-bzw. Phosphoniumfluoride der Formel (I) in Abmischung mit monofunktionellen Alkohol(gemische)en eines (mittleren) Molekulargewichtsbereiches von 32: - 250 g/mol eingesetzt, wobei die Konzentration des quatemären Ammonium- bzw. Phosphoniumfluorides 20 Masse-%, besonders bevorzugt 30 Masse-%, in der Mischung nicht unterschreitet.

Gegenstand der Erfindung ist weiterhin das vorstehend beschriebene Verfahren, dadurch gekennzeichnet, daß als Trimerisierungskatalysator quaternäre Ammonium- bzw. Phosphoniumfluoride der Formel (I) in Abmischung mit organischen Säuren eines pKₛ-Wertes größer als 2 (bestimmt in H₂O bei 25°C) sowie Oxalsäure, wobei das molare Verhältnis von organischer Säure zu Fluoridion, F⁻, den Wert 10, bevorzugt 5, besonders bevorzugt 2 nicht überschreitet.

Art und Ausmaß der Solvatisierung der Fluoridanionen sind von erfindungswesentlicher Bedeutung zur Durchführung des hier beschriebenen Verfahrens.

Ein erfindungswesentliches Merkmal des vorgestellten Verfahrens ist es, den Tetraalkylphosphonium- bzw. -ammoniumfluorid-Katalysator so konzentriert wie möglich bis hin zur Dosierung von praktisch reinem Wirkstoff bei der Trimerisierung einzusetzen. Dem sind in der Praxis Grenzen gesetzt, die aus den Erfordernissen an die technische Handhabung des Katalysators resultieren. So ist die Löslichkeit bzw. schnelle homogene Verteilung beispielsweise fester Katalysatoren bzw. hochkonzentrierter und mitunter höherviskoser Katalysatorlösungen im zu trimerisierenden (Poly)isocyanat(gemisch) mitunter zu gering, um eine spontane Gelpartikelbildung infolge lokaler Übervernetzungen vermeiden zu können.

In diesem Zusammenhang sei deshalb auf eine wesentliche Eigenschaft von **S** hingewiesen: die Komplexierungseigenschaften von S für das Fluoridion müssen so geartet sein, daß, zumindest bis zur homogenen Verteilung im zu trimerisierenden (Poly)isocyanat(gemisch), eine langsame Entfaltung der katalytischen Aktivität des Fluoridanions einsetzt ('slow release' Mechanismus). Anderenfalls kann es zur Ausbildung spontaner Übervernetzungen die in der Bildung trüber, unbrauchbarer Produkte resultiert, kommen. Dem kann zwar durch eine stärkere Verdünnung des Katalysators begegnet werden, wie überraschenderweise gefünden wurde, wird dadurch der Iminooxadiazindionanteil im Trimerengemisch jedoch immer weiter abgesenkt (vgl. Beispiel 4).

So ist die Verwendung flüssiger Tetraalkylammonium- bzw. -phosphoniumfluoride als rimerisierungskatalysator besonders bevorzugt. Es können aber auch als Reinsubstanz feste aber bereits bei Zugabe sehr geringer Mengen an **S** flüssige Verbindungen wie z.B. Tetrabutylammonium- bzw. Tetrabutylphosphoniumfluorid eingesetzt werden.

Mit prinzipiell gleichem Erfolg kann man reine Verbindungen bzw. beliebige Mischungen von Verbindungen der Summenformel R₄E⁺ F⁻ einsetzen, wobei E für N oder P und R für gleiche oder verschiedene, gegebenenfalls verzweigte, gegebenenfalls substituierte, aliphatische, aromatische und/oder araliphatische C₁-C₂₀ Reste steht. Kommerziell erhältlich, zumindest in Form ihrer Salze mit anderen Gegenionen als dem Fluoridion, die sich aber leicht in die Fluoridform überführen lassen, z.B. Chloride, Bromide, Iodide, (Hydrogen)sulfate; vgl. u.a. *J. Org. Chem*. **1989,** *54*, S. 4827-4829, sowie *Synthesis* **1988**, *12* S. 953-5 und Bsp. 1, sind beispielsweise Tetrakis(hydroxymethyl)phosphoniumchlorid und -sulfat, Tetraethyl-, Tetrabutyl-, Tetraoctyl-, Tetrakis(hexadecyl)-, Tributyl(tetradecyl)-, Tributyl(hexadecyl)- und Trioctyl(methyl)ammonium- bzw. -phosphoniumchlorid bzw. -bromid und/oder--iodid.

Auch die Verwendung von Phenyl(alkyl)derivaten ist prinzipiell möglich, wenngleich sie aufgrund ihrer im Vergleich zu den rein aliphatisch substituierten Vertretern schlechteren Löslichkeit in den für den Einsatz in der Isocyanatoligomerisierung geeigneten Solvatisierungs- bzw. Lösungsmitteln, insbesondere monofünktionelle Alkohole, weniger bevorzugt sind.

Ganz allgemein ist die besondere Rolle, die Art und Menge des/der während der Katalyse anwesenden Solvatisierungsmittel(s) **S** zur bevorzugten Bildung von Iminooxadiazindiongruppen zukommt, für den Fachmann aus dem Stand der Technik nicht ersichtlich.

Das molare Verhältnis F⁻: S ist jedoch von entscheidender Bedeutung für die Durchführung des erfindungsgemäßen Verfahrens um zu einem hohen Gehalt an Iminooxadiazindiongruppen, d.h. mindestens 30 mol-% im Trimerengemisch, zu gelangen.

Durch die überraschende Beobachtung, daß die Selektivität der Katalyse signifikant von der Konzentration des Katalysators abhängt, hebt sich die vorliegende Erfindung von älteren Veröffentlichungen die den Einsatz von Fluoriden zur Isocyanat-Trimerisierung unter Isocyanuratbildung, (mit)beschreiben in entscheidender Weise ab.

So wird in der DE-A 38 27 596 auf die Möglichkeit hingewiesen, mit quaternären Ammonium- sowie Phosphoniumfluoriden Isocyanuratgruppen aufweisende Polyisocyanate herzustellen. In der genannten Patentschrift wird auf S. 3, Zeilen 30-35 explizit darauf verwiesen, daß die Fluoridkonzentration der zur homogenen Katalyse einzusetzenden Lösung 0,5 mMol F⁻ je g Lösung nicht überschreiten sollte, wobei als Lösungsmittel z.B. 2-Ethyl-1,3-hexandiol, Acetonitril oder N,N-Dimethylformamid (DMF) einzusetzen sind.

Eigene Versuche, nach der in der DE-A 38 27 596 vorgeschlagene Verfahrensweise durchgeführt, zeigen allerdings, daß der Einsatz dieser Fluoride, gelöst in den genannten Lösungsmitteln, in der in den vorstehend genannten Anmeldungen angeführten bevorzugten Fluoridionenkonzentration (0,01-0,1 mMol F⁻ je g Lösung, d.h. ca. 0,02-0,2 % F⁻ in der Katalysatorlösung), nur zur Bildung extrem trüber Produkte führt, die als hochwertige Isocyanatkomponenten z.B. für die Herstellung von Polyurethanlacken und -beschichtungsmitteln völlig unbrauchbar sind. Der Iminooxadiazindionanteil im Trimerengemisch so hergestellter Produkte ist zudem gering und wird bei stärkerer Verdünnung der Katalysatorlösung mit den o.g. Lösungsmitteln weiter verringert (Beispiel 2, Tabelle 1).

Aprotische Katalysatorlösungsmittel wie Acetonitril oder DMF sind für das beanspruchte Verfahren generell wenig geeignet, da sie bei der, in der Regel im Anschluß an die (anteilige) Trimerisierung der Isocyanatgruppen, beispielsweise von Diisocyanaten wie HDI, erfolgende Rückgewinnung des nicht umgesetzten Monomers, z.B. durch Destillation, mit diesem aus dem Prozeß entfernt und anschließend, wiederum mit diesem gemeinsam, erneut eingesetzt werden wobei sie sich bei fortlaufender Wiederholung dieses Procederes immer weiter anreichern würden was früher oder später ihre aufwendige Abtrennung vom 'im Kreis gefahrenen' Monomer erforderlich machen würde. Ein solches Verfahren wäre natürlich u.a. unter ökonomischen Gesichtspunkten mit gravierenden Mängeln behaftet.

Weiterhin kann DMF mit Isocyanaten bei erhöhter Temperatur unter Bildung unerwünschter Nebenprodukte reagieren (vgl. *Angew. Makromol. Chem*., **1992**, *197*, 131-139, CO₂-Entwicklung und Formamidinbildung) und in Kombination mit basischen Stoffen (z.B. Verunreinigungen) bei niedriger Temperatur die unerwünschte Linearpolymerisation der (Poly)isocyanate unter Bildung äußerst schwerlöslicher 1-Nylonverbindungen katalysieren (z.B. 'Organic Chemistry, A Series of Monographs', Bd. 13 B/ 2, Acad. Press New York u. London 1972, S. 332 ff und darin zit. Literatur).

Hinzu kommt, daß bei Einsatz aprotischer Katalysatorlösungsmittel wie Acetonitril und DMF auch bei Verwendung relativ stark verdünnter Katalysatorlösungen extrem trübe, sehr grobe, gelartige Feststoffpartikel enthaltende Trimerisat-Rohlösungen resultieren, die erst nach aufwendigen Filtrationsschritten weiter aufgearbeitet werden können und selbst dann keine völlig trübungsfreien Harze nach Aufarbeitung durch Dünnschichtdestillation liefern (vgl. Beispiele 2b und 2c). Der Iminooxadiazindionanteil in so hergestellten Trimerisatharzen ist zudem sehr gering (Tabelle 1).

Ferner findet sich in den Ausführungsbeispielen der DE-A 38 27 596 lediglich ein Hinweis auf die Herstellung eines Phosphoniumfluorid-Katalysators, der zusätzlich auf einem festen Trägermaterial (Kieselgel) aufgezogen worden ist (Seite 6, Tab. 1, Bsp. 4). Sein Einsatz zur Isocyanat-Modifizierung wird in der genannten Patentschrift nicht erwähnt.

Ähnliche Katalysatorsysteme, hier in Verbindung mit CO₂, und deren Einsatz zur Herstellung von modifizierten, Isocyanuratgruppen aufweisenden Polyisocyanaten werden in der DE-A 39 02 078 beschrieben, wenngleich Phosphoniumsalze an mehreren Stellen dieser Patentschrift ausdrücklich gegenüber Ammoniumspezies als 'weniger bevorzugt' herausgestellt werden (Seite 3, Zeilen 32/33, Zeilen 60/61, Seite 4, Zeile 12). Die Aussagen zu bevorzugten Katalysatorkonzentrationen bei der Homogenkatalyse sind analog zu den schon in der DE-A 38 27 596 getroffenen. In den Ausführungsbeispielen der genannten Patentschrift finden sich weiterhin keinerlei Hinweise auf die Herstellung oder Verwendung von Phosphoniumfluoriden als Katalysator für die Isocyanatmodifizierung. Auch wird in der genannten Patentschrift ausdrücklich darauf verwiesen, daß in den resultierenden Produkten 'der Iminooxadiazindionanteil von untergeordneter Größe' bleibt (S. 4, Zeilen 51-52). Die Ausführungsbeispiele 6 bis 9 der DE-A 39 02 078, in denen über die anteilige Bildung von Iminooxadiazindionen neben Isocyanurat und Oxadiazintrion, als den beiden Hauptprodukten der Reaktion, berichtet wird, legen zudem eher den Schluß nahe, daß es einerseits zur Iminooxadiazindionbildung der Gegenwart von CO₂ bei der Trimerisierungsreaktion bedarf und daß es sich andererseits bei den Imonooxadiazindionen eher um unerwünschte Nebenprodukte handelt.

Generell ist weder der DE-A 38 27 596 noch der DE-A 39 02 078 ein Hinweis auf die besondere Rolle zu entnehmen, die das Katalysatorlösungsmittel nicht nur schlechthin zur Verdünnung des Katalysators spielt, sondern sowohl zur Gewährleistung eines problemlosen Reaktionsverlaufes, d.h. zur Vermeidung von Trübungs- bzw. Feststoffbildung, als auch als selektivitätssteuerndes Agenz (Solvatisierungsmittel) zur bevorzugten Bildung von Iminooxadiazindiongruppen bei der Isocyanattrimerisierung hat.

Weiterhin finden sich in der (Patent)literatur Hinweise auf die Möglichkeit einer Verwendung von Phosphoniumfluoriden, gegebenenfalls 'in situ' aus einem Alkali- oder Erdalkalimetallfluorid und einem anderen quaternären Phosphoniumsalz (Chlorid, Bromid etc., vgl. weiter oben) generiert, für die Isocyanatmodifizierung (Phasentransferkatalyse, z.B. *Isr. J. Chem.,* **1985**, *26*, 222-224, der Einsatz von Phosphoniumfluoriden wird dort allerdings nicht beschrieben).

Diesem Gedanken folgend werden in der EP-A 0 315 692 Kaliumfluorid-katalysierte Prozesse zur Herstellung von Substanzen mit Isocyanurat-Ringstrukturen beschrieben, wobei u.a. die simultane Anwesenheit von Onium-Verbindungen zur 'Steigerung der Effizienz der Reaktion' vorgeschlagen wird. In den Ausführungsbeispielen dieser Patentschrift finden sich allerdings keinerlei Hinweise auf den Einsatz von Phosphoniumsalzen. Außerdem wird hauptsächlich auf die Trimerisierung aromatischer Isocyanate (TDI, MDI) eingegangen und nur anhand der Reaktion von n-Butylisocyanat mit KF an zwei Beispielen die katalytische Wirkung des Kaliumfluorides an sich (Bsp. 1 der EP-A 0 315 692) bzw. in Gegenwart von quaternärem Ammoniumsalz (Benzyltrimethylammoniumchlorid, Bsp. 5 der EP-A 0 315 692) zur Trimerisierung von Isocyanaten mit aliphatisch gebundenen NCO-Gruppen unter Isocyanuratbildung demonstriert. Die hohe Reaktionstemperatur (120°C) sowie die vergleichsweise langen Reaktionszeiten (8 h im Bsp. 1, 4 h im Bsp. 5 der EP-A 0 315 692) bei hoher Katalysatorkonzentration, die technisch unvorteilhafte Abtrennung der festen Kaliumsalzkomponenten nach der Reaktion durch Filtration (Bsp. 1 der EP-A 0 315 692) bzw. Auswaschen mit Wasser (Bsp. 5 der EP-A 0 315 692), letzteres wäre ohnehin prohibitiv für die Herstellung von Isocyanatgruppen enthaltenden Produkten, sowie der Umstand, daß durch die (mit)beanspruchte Verwendung von Oniumsalz neben Kaliumfluorid eine ständige 'Extraktion' von Fluoridionen aus der unlöslichen anorganischen Phase, die als eigentlicher Katalysator bezeichnet wird, in die organische, Isocyanat enthaltende Phase erfolgt, lassen die Methode generell als wenig vorteilhaft und technisch kaum realisierbar erscheinen.

Bei der aus der EP-A235 388 bekannten Umsetzung von Isocyanaten mit Carbonsäure(anhydride)n unter Fluoridkatalyse werden die entsprechenden Polyamide/-imide erhalten, aber keine Produkte einer NCO/NCO-Reaktion.

In keiner der letztgenannten Schriften findet sich ein Hinweis auf die (Mit)entstehung von Iminooxadiazindionstrukturen neben den beschriebenen Isocyanuraten.

Es war deshalb für den Fachmann auf der Basis des vorbeschriebenen Standes der Technik nicht ersichtlich, daß im organischen Medium (in der Regel das zu modifizierende Isocyanat(gemisch)) vollständig lösliche, quaternäre Ammonium- bzw. -phosphoniumfluoride oder spezielle Kombinationen dieser Phosphoniumfluoride mit bestimmten Solvatisierungsmitteln für das Fluoridanion der in der vorliegenden Erfindung näher beschriebenen Art besonders vorteilhaft zur Herstellung trübungsfreier Isocyanattrimerisatharze mit hohem Iminooxadiazindiongruppenanteil im Trimerengemisch geeignet sein könnten.

Nach dem erfindungsgemäßen Verfahren können als protische Solvatisierungsmittel **S** Wasser, Alkohole und/oder aliphatische sowie aromatische Carbonsäuren, wie weiter unten ausführlich diskutiert wird, eingesetzt werden. Allerdings ist die Menge an jeweils zuzusetzendem **S** zur Erzielung eines möglichst hohen Gehaltes an Iminooxadiazindionen nach oben hin begrenzt, d.h. bei sukzessiver Absenkung der Konzentration an quaternärem Ammonium- bzw. -phosphoniumfluorid in der Katalysatormischung verliert dieses zunehmend seine Selektivität für die bevorzugte Iminooxadiazindionbildung und es entstehen, neben den Folgeprodukten die aus der Anwesenheit von **S** resultieren, im wesentlichen nur die lange bekannten Isocyanurate.

Geeignete Monoalkohole sind geradkettige und verzweigte, primäre, sekundäre und tertiäre Alkohole mit ein bis zwanzig C-Atomen, bevorzugt ein bis acht C-Atomen, z.B. Methanol, Ethanol, n- sowie iso-Propanol, 1- und 2-Butanol, iso-Butanol und 2-Ethylhexanol.

Geeignete organische Säuren sind allgemein Oxalsäure sowie schwächere Säuren, die einen pK_{S}-Wert oberhalb 2,0 aufweisen, wie z.B. Ameisensäure, Essigsäure, (gegebenenfalls durch Hydroxigruppen substituierte) Pivalinsäure(n), (gegebenenfalls an der/den CH₂-Gruppe(n) substituierte) Malon-, Bernstein- sowie Propan-1,3-dicarbonsäure(n), ferner Phthalsäure, Salicylsäure etc. Als Tabellierungsbedingung für den pK_{S}-Wert wird im allgemeinen Wasser bei 25°C angegeben (vgl. auch Beispiel 5).

Das erfindungsgemäße Verfahren kann in einem Temperaturbereich von ca. 20°C (Zimmertemperatur) bis ca. 200°C, bevorzugt zwischen 30°C und 120°C, besonders bevorzugt zwischen 40°C und 100°C unter anteiliger Umsetzung der beteiligten Isocyanatgruppen des/der Ausgangs(poly)isocyanat(es)/mischung erfolgen, wobei der Umsetzungsgrad U_{NCO}, berechnet als Quotient aus der Differenz des NCO-Gehaltes des/der Ausgangs(poly)isocyanat(es)/mischung vor der Trimerisierung und des NCO-Gehaltes der Reaktionsmischung nach Abbruch der Reaktion und dem NCO-Gehalt des/der Ausgangs(poly)isocyanat(es)/mischung vor der Trimerisierung, zwischen 5 % und 60 %, bevorzugt zwischen 10 % und 40 % liegt.

Dabei nicht umgesetztes Monomer kann nach Desaktivierung des Katalysatorsystems, z.B. durch (Dünnschicht)destillation oder Extraktion, abgetrennt und anschließend wiederverwendet werden.

Zur Desaktivierung des Katalysatorsystems nach Erreichen des gewünschten U_{NCO} eignen sich prinzipiell alle vorbeschriebenen Methoden des Standes der Technik, wie sie beim Abstoppen der Trimerisierungsreaktion unter Isocyanuratbildung angewandt werden. Das sind z.B. die Zugabe unter- oder auch überstöchiometrischer Mengen an starken Säuren oder Säurederivaten (z.B. Benzoylchlorid, saure Ester der phosphorigen Säure und der Phosphorsäure, diese Säuren selbst etc., nicht jedoch HF und andere schwache Säuren eines pKₛ-Wertes oberhalb 2,0), adsorptive Bindung des Katalysators und anschließende Abtrennung durch Filtration, thermische Desaktivierung u.s.w.

Die Entfernung von überschüssigem/n Ausgangsisocyanat/en, sofern es sich bei diesem/n um (ein) niedermolekulare(s), 'monomere(s)' (Di)isocyanat(e) handelt, erfolgt vorzugsweise, wenn die erfindungsgemäßen Verfahrensprodukte für Anwendungen auf dem Polyurethanlack- und beschichtungsmittelsektor bestimmt sind. Hierbei profitiert man von der exellenten Farbzahl und -stabilität der Verfahrensprodukte sowie ihrer hohen Rückspaltstabilität in das/die zugrundeliegende(n) monomere(n) (Di)isocyanat(e).

Zur erfindungsgemäßen Herstellung der Trimeren(mischungen) genügen Katalysatorkonzentrationen, bezogen auf eingesetzte(s) (Poly)isocyanat(mischung) und das Fluoridion (rel. Molmasse 19), zwischen 1 ppm und 1 %, bevorzugt zwischen 1 ppm und 0,1%, besonders bevorzugt zwischen 1 ppm und 0,05 %.

Nach einer besonderen, kontinuierlich arbeitenden Ausführungsform des erfindungsgemäßen Verfahrens wird die Oligomerisierung in einem Rohrreaktor vorgenommen.

Hierbei profitiert man von der geringeren Neigung insbesondere der Phosphoniumfluoridkatalysatoren, trotz Applikation in hochkonzentrierter Lösung bzw. als reiner Wirkstoff, spontan Gelteilchen im Produkt zu bilden. Zudem können wiederum höherkonzentrierte Katalysatorlösungen verwendet werden als bei diskontinuierlichen ('batch'-) Trimerisierungen, da die Vermischungsgeschwindigkeiten in Rohrreaktoren mit turbulenter Pfropfenströmung gegenüber der Vermischung der Reaktanden in Rührkesseln erheblich beschleunigt ist, der o.g. 'slow release'-Mechanismus mithin deulich weniger lange anhalten muß.

Das erfindungsgemäße Verfahren kann sowohl lösungsmittelfrei als auch unter Verdünnung des Ausgangs(poly)isocyanates bzw. der Ausgangs(poly)isocyanat(mischung)e(n) durchgeführt werden. Zur Verdünnung eignen sich hierbei alle, gegenüber NCO-Gruppen inerte organische Verbindungen wie Toluol, Xylol(e), höhere Aromaten, Ester, Ether, Ketone, C₁₂ -C₂₀-Alkylsulfonsäureester sowie Gemische derartiger Lösungsmittel.

Zur Durchführung des erfindungsgemäßen Verfahrens können alle Di- oder Polyisocyanate mit einem (mittleren) Molekulargewicht von 140 - 600 g/mol mit unabhängig voneinander aliphatisch, cycloaliphatisch und/oder araliphatisch gebundenen Isocyanatgruppen in reiner Form oder als beliebige Mischungen untereinander verwendet werden. Beispielhaft seien genannt:
Hexamethylendiisocyanat (HDI), 2-Methylpentan-1,5-diisocyanat (MPDI); 1,3-Bis(isocyanatomethyl)cyclohexan (1,3-H₆-XDI), 3(4)-Isocyanatomethyl-1-methylcyclohexylisocyanat (IMCI); Isophorondiisocyanat (IPDI), Bis(isocyanatomethyl)-norbornan (NBDI), 4-Isocyanatomethyl-1,8-octandiisocyanat (Triisocyanatononan, TIN), 1,3-Bis(isocyanatomethyl)benzol, 1,3-Bis(2-isocyanatopropyl-2)benzol und Bis(4(2)-Isocyanatocyclohexyl)methan (H₁₂MDI, Desmodur® W, Produkt der Bayer AG). Hierbei ist es belanglos, nach welchen Verfahren die vorstehend genannten (Poly)isocyanate hergestellt werden, d.h. mit oder ohne Verwendung von Phosgen.

Bevorzugt werden HDI, MPDI, 1,3-H₆XDI, NBDI sowie Mischungen aus HDI und IPDI eingesetzt.

Es kann weiterhin von Vorteil sein, Gemische bestimmter (Poly)isocyanate im erfindungsgemäßen Verfahren einzusetzen, beispielsweise um dem Anforderungsprofil des jeweiligen Produktes bzw. Produktgemisches optimal zu entsprechen. So werden in vielen Anwendungen, beispielsweise bei der Automobil(erst)lackierung, Polyisocyanatgemische auf Basis, gegebenenfalls verzweigter, linearaliphatischer, z.B. HDI, einerseits und cycloaliphatischer Diisocyanate, z.B. IPDI oder H₁₂MDI (Desmodur® W-Handelsprodukt der Bayer AG), andererseits eingesetzt. Diese Gemische werden in der Regel durch nachträgliches Abmischen von Polyisocyanaten auf Basis von Diisocyanaten des einen mit denen des anderen Typs hergestellt. Es kann aber auch von Vorteil sein, sie durch simultane Mischtrimerisation aus dem entsprechenden Gemisch der monomeren Komponenten herzustellen (EP-A 00 47 452). Viele Polyisocyanate auf Basis cycloaliphatischer Diisocyanate des Standes der Technik sind fest. Sie weisen zuweilen eine derart hohe Schmelzviskosität auf, daß mitunter schon die Monomerenabtrennung durch (Dünnschicht)destillation erhebliche Schwierigkeiten bereitet. Deshalb bedarf es zu ihrer Verarbeitung bzw. mitunter auch bereits bei der Dünnschichtdestillation der Verwendung von Lösungsmitteln bzw. von Fließhilfsmitteln. Will man keine allzu großen Einbußen in Umsetzungsgrad (Harzausbeute) und NCO-Funktionalität bei der Herstellung dieser Polyisocyanate hinnehmen, sind Lösungskonzentrationen um 70 % (Fest)harz, z.B. bei Isocyanurat-Polyisocyanaten auf Basis cycloaliphatischer Diisocyanate, bei gut verarbeitbaren dynamischen Viskositäten zwischen ein und zehn Pa·s (gemessen bei 23°C) marktüblicher Stand der Technik.

Trimerisiert man hingegen Gemische linearaliphatischer, z.B. HDI, und cycloaliphatischer Diisocyanate, z.B. IPDI, gemäß dem erfindungsgemäßen Verfahren unter (teilweiser) Iminooxadiazindionbildung, erhält man auch bei Zimmertemperatur fließfahige Produkte (Viskosität bei 23°C unter 100 Pa·s) die zudem bei sukzessiver Lösungsmittelzugabe eine drastisch schnellere Viskositätsabnahme aufweisen, als Produkte entsprechender Zusammensetzung (NCO-Funktionalität, Diisocyanatbasis, mittleres Molekulargewicht) des Standes der Technik (Beispiel 6).

Die nach dem erfindungsgemäßen Verfahren erhseltenen Produkte bzw. Produktgemische stellen mithin vielseitig verwendbare Ausgangsmaterialien zur Herstellung von, gegebenenfalls geschäumte(n), Kunststoffe(n) sowie Lacken, Beschichtungsmitteln, Klebstoffen und Zuschlagstoffen dar.

Vor ihrer Verwendung als Isocyanat-Komponente in Polyurethansystemen können die Verfahrensprodukte gegebenenfalls an den Isocyanatgruppen weiter modifiziert werden, beispielsweise durch Einführung von Urethan-, Harnstoff-, Biuret- und/oder Allophanatgruppen oder durch eine teilweise oder vollständige Umsetzung der NCO-Gruppen mit speziellen, wieder abspaltbaren Verbindungen ('Blockierung'). Hierfür eignen sich beispielsweise Phenole, Lactame wie z.B. ε-Caprolactam, Oxime, Di- und Triazole, bestimmte Amine wie z.B. Diiso-Propylamin, CH-acide Verbindungen wie z.B. Malonsäuredialkylester, Acetessigester etc.

Insbesondere zur Herstellung von, gegebenenfalls wasserdipergierbaren Ein- und Zweikomponenten-Polyurethanlacken eignen sie sich, gegebenenfalls in NCOblockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende, im wesentlichen Isocyanuratgruppen enthaltende Produkte des Standes der Technik. Ihre Resistenz gegenüber der Einwirkung von Luftfeuchtigkeit (z.B. Hautbildung im offenen Gebinde, mattes Erscheinungsbild von Flächen, die bei hoher Luftfeuchtigkeit und Umgebungstemperatur lackiert wurden, sog. 'downglossing') ist ebenfalls gegenüber den (fast) ausschließlich Isocyanuratgruppen enthaltenden Produkten verbessert.

### Beispiele

Alle Prozentangaben sind, soweit nicht anders vermerkt, als Gewichtsprozent zu verstehen.

Mol-% Angaben werden NMR-spektroskopisch ermittelt und beziehen sich immer, so nicht anders ausgewiesen, auf die Summe der durch die Modifizierungsreaktion ('Trimerisierung') entstehenden NCO-Folgeprodukte. Die Messungen erfolgen auf dem Gerät DPX 400 der Fa. Bruker an ca. 5 %igen (¹H-NMR) bzw. ca. 50 %igen (¹³C-NMR) Proben in trockenem CDCl₃ bei einer Frequenz von 400 MHz (¹H-NMR) bzw. 100 MHz (¹³C-NMR). Als Referenz für die ppm-Skale werden geringe Mengen von Tetramethylsilan im Lösungsmittel mit einer ¹H-chem. Verschiebung von 0 ppm (¹H-NMR) bzw. das Lösungsmittel selbst (CDCl₃) mit einer Verschiebung von 77,0 ppm (¹³C-NMR) gewählt. Daten für die chemische Verschiebung der in Frage kommenden Verbindungen sind der Literatur entnommen (vgl. Die Angewandte Makromolekulare Chemie 141, 1986, 173-183 und darin zit. Lit) bzw. durch Vermessung von Modellsubstanzen gewonnen worden. Das in Anlehnung an das in *Ber. d*. *dtsch. Chem. Ges.* **1927**, *60*, 295 beschriebene Verfahren aus Methylisocyanat unter Katalyse mit ca. 3 % Tri-n-butylphosphin in ca. 70 %iger Ausbeute zugängliche 3,5-Dimethyl-2-methylimino-4,6-diketo-1,3,5-oxadiazin weist folgende NMR-chem. Verschiebungen auf (in ppm): 3,09, 3,08 und 2,84 (¹H-NMR, CH₃) bzw. 148,3, 144,6 und 137,3 (¹³C-NMR, C=O/C=N). Die erfindungsgemäßen Verfahrensprodukte mit Iminooxadiazindionstruktur haben sehr ähnliche ¹³C-NMR chem. Verschiebungen der C=O/C=N Atome und sind zweifelsfrei als solche von anderen Isocyanat-Folgeprodukten zu unterscheiden.

Die dynamischen Viskositäten werden bei 23°C mit dem Viskosimeter VT 550 der Fa. Haake bestimmt. Durch Messungen bei unterschiedlichen Schergeschwindigkeiten ist sichergestellt worden, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Restmonomergehalte erfolgt gaschromatographisch.

Die Trübung der Trimerisatharze wird mit einem Gerät der Fa. Hach ermittelt. Hierzu werden Streulichtmessungen in 90° zur Richtung eines durch die Harzprobe geleiteten Lichtstrahles der Wellenlänge 400 - 800 nm durchgeführt und in Einheiten bezogen auf Formazin-Standardlösungen, TE(F), angegeben.

Der überwiegende Teil der Reaktionen wird beispielhaft mit HDI als zu trimerisierendem Isocyanat und Katalysatoren auf Basis Tetrabutylphosphoniumfluorid unter einer Stickstoffatmosphäre durchgeführt. Das geschieht nur zur Verdeutlichung der Vorteile des erfindungsgemäßen Verfahrens und soll keine Einschränkung der vorliegenden Erfindung auf die beschriebenen Systeme bzw. Reaktionsbedingungen bedeuten.

### Beispiel 1: Herstellung von quaternären Phosphoniumfluoriden (Stammlösungen)

in Anlehnung an *J. Org. Chem.* **1989**, *54*, S. 4827-4829. (Vorschriften für R₄N⁺Cl⁻ wurden analog auf R₄P⁺Cl⁻ umgearbeitet)
a) BU₄P⁺ F⁻ in Methanol/iso-Propanol (Stammlösung 1a)
953,8 g einer 71,4 %igen Bu₄P⁺ Cl⁻-Lösung in iso-Propanol (Cyphos® 443P, Produkt der Fa. Cytec), entsprechend 2,3 mol Bu₄P⁺ Cl⁻ werden in 1 kg techn. Methanol (ca. 0,2 % H₂O) gelöst, mit 150 g (2,58 mol) pulverisiertem Kaliumfluorid versetzt und 24 h bei 20-25°C (Zimmertemperatur) gerührt. Anschließend wird filtriert, der Filterrückstand mit 2 mal 100 g techn. Methanol gewaschen und die vereinigten Filtrate erneut mit 150 g (2,58 mol) pulverisiertem Kaliumfluorid versetzt und 24 h bei 20-25°C (Zimmertemperatur) gerührt. Nach anschließender Filtration und erneutem Waschen mit 2 mal 100g techn. Methanol wird bei maximal 30°C und einem Druck von ca. 1 mbar am Rotationsverdampfer von überschüssigem Methanol und iso-Propanol weitgehend befreit und erneut filtriert. Die so erhaltene, nahezu farblose Lösung weist folgende Daten auf:

| | |
|---|---|
| Fluorid (mit ionensensitiver Elektrode bei pH 5,5) | 5,0 % |
| Chlor (gesamt, nach Aufschluß, gravimetrisch) | 0,4 % |
| MeOH (gaschromatografisch, nach Normierung) | 16,3 % |
| *i*-PrOH (gaschromatografisch, nach Normierung) | 7,3 % |

b) Bu₃(C₁₄H₂₉)P⁺ F⁻ in Methanol/iso-Propanol (Stammlösung 1b)
500 g einer 74,2 %igen Bu₃(C₁₄H₂₉)P⁺ Cl⁻-Lösung in iso-Propanol (Cyphos® 3453P, Produkt der Fa. Cytec), entsprechend 0,85 mol Bu₃(C₁₄H₂₉)P⁺ Cl⁻ werden in 0,5 kg techn. Methanol (ca. 0,2 % H₂O) gelöst, mit 50 g (0,86 mol) pulverisiertem Kaliumfluorid versetzt und 24 h bei 20-25°C (Zimmertemperatur) gerührt. Anschließend wird filtriert, der Filterrückstand mit 2 mal 50 g techn. Methanol gewaschen, die vereinigten Filtrate erneut mit 50 g (0,86 mol) pulverisiertem Kaliumfluorid versetzt und 24 h bei 20-25°C (Zimmertemperatur) gerührt. Nach anschließender Filtration und erneutem Waschen mit 2 mal 50 g techn. Methanol wird bei maximal 30°C und einem Druck von ca. 1 mbar am Rotationsverdampfer von überschüssigem Methanol und iso-Propanol weitgehend befreit und erneut filtriert. Die so erhaltene Lösung weist folgende Daten auf:

| | |
|---|---|
| Fluorid (mit ionensensitiver Elektrode bei pH 5,5) | 3,65 % |
| Chlor (gesamt, nach Aufschluß, gravimetrisch) | 0,145 % |
| MeOH (gaschromatografisch, nach Normierung) | 9,1 % |
| *i*-PrOH (gaschromatografisch, nach Normierung) | 3,8 % |

c) Ph₃(Bu)P⁺ F⁻ in Methanol (Stammlösung 1c)
20 g (56,3 mmol) Ph₃(Bu)P⁺ Cl⁻ (Fa. Chemconserve) werden in 40 g techn. Methanol (ca. 0,2 % H₂O) gelöst, mit 3,3 g (56,8 mmol) pulverisiertem Kaliumfluorid versetzt und 24 h bei 20-25°C (Zimmertemperatur) gerührt. Anschließend wird filtriert, der Filterrückstand mit 2 mal 5 g techn. Methanol gewaschen und die vereinigten Filtrate erneut mit 3,3 g (56,8 mmol) pulverisiertem Kaliumfluorid versetzt und 24 h bei 20-25°C (Zimmertemperatur) gerührt. Nach anschließender Filtration und erneutem Waschen mit 2 mal 5 g techn. Methanol wird bei maximal 30°C und einem Druck von ca. 1 mbar am Rotationsverdampfer bis zur beginnenden Kristallisation von überschüssigem Methanol befreit und erneut filtriert. Dabei ist darauf zu achten, daß nur von Kaliumsalzen, die infolge weiterer Aufkonzentrierung der Lösung ausfallen, abgetrennt wird und kein Phosphoniumsalz im Filterrückstand verbleibt (Löslichkeitsprobe). Die so erhaltene Lösung weist folgende Daten auf:

| | |
|---|---|
| Fluorid (mit ionensensitiver Elektrode bei pH 5,5) | 3,15 % |
| Chlor (gesamt, nach Aufschluß, gravimetrisch) | <0,2 % |
| MeOH (gaschromatografisch, nach Normierung) | 42,8 % |

d) R₃(Me)N⁺F⁻ in Methanol/iso-Propanol (Stammlösung 1d)
151,3 g einer ca. 90 %igen R₃(Me)N⁺Cl⁻-Lösung in iso-Propanol (Adogen® 464, Produkt der Fa. Aldrich, R steht für C₈-C₁₀-Reste wobei C₈ überwiegt, Chlorgehalt: 7,1 %) werden in 170 g techn. Methanol (ca. 0,2 % H₂O) gelöst, mit 17,6 g pulverisiertem Kaliumfluorid versetzt und 24 bei 20 bis 25°C (Zimmertemperatur) gerührt. Anschließend wird filtriert, der Filterrückstand mit 2 mal 100 g techn. Methanol gewaschen und die vereinigten Filtrate erneut mit 17,6 g pulverisiertem Kaliumfluorid versetzt und 24 h bei 20 bis 25°C (Zimmertemperatur) gerührt. Nach anschließender Filtration und erneutem Waschen mit 2 mal 100 g techn. Methanol wird bei maximal 25°C und einem Druck von ca. 1 mbar am Rotationsverdampfer von überschüssigem Methanol und iso-Propanol bis zur Gewichtskonstanz befreit und erneut filtriert. Die so erhaltene, schwach gelb gefärbte Lösung weist folgende Daten auf:

| | |
|---|---|
| Fluorid (mit ionensensitiver Elektrode bei pH 5,5) | 3,4 % |
| Chlor (gesamt, nach Aufschluß, gravimetrisch) | 0,2 % |
| MeOH (gaschromatografisch, nach Normierung) | 13,9 % |
| *i*-PrOH (gaschromatografisch, nach Normierung) | 2,5 % |

### Beispiel 2: Vergleichsbeispiele (nicht erfindungsgemäß)

a) HDI-Trimerisierung mit einer ca. 1,5 %igen Tetrabutylphosphoniumfluoridlösung in 2-Ethyl-1,3-hexandiol (ca. 0,1%ig an F⁻, Vorzugsbereich der Katalysatorkonzentration gemäß DE-A 38 27 596 bzw. DE-A 39 02 078). 200 g (1,19 mol) HDI werden in einer Dreihalskolben-Rührapparatur bei 60°C zunächst durch ca. einstündiges Rühren im Vakuum (0,1 mbar) von gelösten Gasen befreit, mit Stickstoff belüftet und anschließend wird im Verlauf von 4 h durch tropfenweise Zugabe einer mit 2-Ethyl-1,3-hexandiol auf ca. 0,1% F⁻ verdünnten Lösung der Bu₄P⁺ F⁻-Stammlösung 1a die Trimerisierungsreaktion bis zu einem NCO-Gehalt der Rohlösung von 42,1 % geführt (Katalysatorbedarf: 46 ppm F⁻, Abstoppen mit 103 mg Dibutylphosphat). Im Verlauf der Katalysatorzugabe bilden sich zunehmend Feststoffpartikel, die sich insbesondere an der Kolbenwandung über der Flüssigkeit ablagern. Das nach Filtration der Rohlösung über ein Faltenfilter und anschließende Dünnschichtdestillation in einem Labor-Dünnschichtverdampfer, Typ Kurzwegverdampfer, bei 140°C/0,2 mbar gewonnene Harz weist die in Tabelle 1 aufgeführten Daten auf.

Wird anstelle des 2-Ethyl-1,3-hexandiols beispielsweise Glykol als Katalysatorlösungsmittel verwendet, resultieren prinzipiell analoge Effekte: zu hohe Trübung der Trimerisat-Rohlösungen und -harze bei tendenziell immer niedrigerer Iminooxadiazindion-Bildungsrate wenn der Katalysator verdünnt wird.
b) HDI-Trimerisierung mit einer ca. 1,5 %igen Tetrabutylphosphoniumfluoridlöung in Acetonitril (ca. 0,1 %ig an F⁻, Vorzugsbereich der Katalysatorkonzentration gemäß DE-A 38 27 596 bzw. DE-A 39 02 078)
c) HDI-Trimerisierung mit einer ca. 1,5 %igen Tetrabutylphosphoniumfluoridlöung in DMF (ca. 0,1%ig an F⁻, Vorzugsbereich der Katalysatorkonzentration gemäß DE-A 38 27 596 bzw. DE-A 39 02 078)

Es wird in völliger Analogie zur Verfahrensweise in Beispiel 2a verfahren. Dabei resultieren trübe und ca. 3 % aprotisches Lösungsmittel enthaltende Rohlösungen, die nach aufwendiger Filtration (gelartige Feststoffpartikel !) und Dünnschichtdestillation stärker getrübte Harze liefern (Tabelle 1). Das Verfahren wäre aufgrund der komplizierten Abtrennung des Katalysatorlösungsmittels ohnehin sehr aufwendig und deshalb kaum realisierbar.

Vgl. auch die unter Beispiel 4 getroffenen Aussagen zur Katalysatordosierung mittels anderer Dosierungstechniken ('Eindüsen').

**Tabelle 1**

| Vergleichsbeispiele zur Katalyse mit quaternären Phosphoniumfluoriden (nicht erfindungsgemäß) | | | |
|---|---|---|---|
| Versuch Nr. | Katalysator | Harztrübung [TE(F)] | Anteil Iminooxadiazindion im Trimerengemisch des Harzes [mol-%] |
| 2a | Bu₄P⁺ F⁻ | 6,6 | 8 |
| 2b | Bu₄P⁺ F⁻ | 4,3 | 22 |
| 2c | Bu₄P⁺ F⁻ | 3,1 | 17 |

### Beispiel 3: Verwendung von Wasser als Solvatisierungsmittel S

Stammlösung 1a, b, c bzw. d wird jeweils mit einem Äquivalent Wasser, bezogen auf den Fluoridgehalt, versetzt und für HDI-Trimerisierungen in der unter 2a beschriebenen Weise verwendet. Dabei wird fortlaufend das im jeweils vorangegangenen Versuch zurückgewonnene Monomer, um die im vorangegangenen Versuch als Harz entnommene HDI-Menge ergänzt, erneut trimerisiert (Versuche 3-1 bis 3-3, jeweils a-d). Es sind bei keinem dieser Versuche Ausflockungen oder Feststoffbildungen während der Reaktion zu beobachten, die isolierten Harze weisen ein sehr niedriges Trübungsniveau und einen hohen Iminooxadiazindiongehalt auf (vgl. Tabelle 2). Der U_{NCO} liegt jeweils bei ca. 20 %. Das Abstoppen der Weiterreaktion erfolgt durch Zugabe der dem F⁻-Verbrauch ensprechenden molaren Menge Dibutylphosphat. Der F⁻-Bedarf der Reaktion liegt zwischen 10 und 30 ppm, bezogen auf die Masse an eingesetztem HDI und die relative Masse des Fluoridions, 19 g/mol.

Wird die H₂O-Menge im verwendeten Katalysator auf bzw. 10 Äquivalente je Äquivalent F⁻ in der Stammlösung 1a erhöht (Versuche 3-4 und 3-5), wird der Iminooxadiazindionanteil in der Trimerenmischung sukzessive abgesenkt. Auch lassen sich bei Versuch 3-5 bereits die aus der NCO-H₂O-Reaktion resultierenden, höherviskosen HDI-Folgeprodukte NMR-spektroskopisch im Harz gut nachweisen (hauptsächlich Biuret und Oxadiazintrion, letzteres entsteht durch sofortigen, fluoridkatalysierten Einbau des bei der NCO-H₂O-Reaktion freiwerdenden Kohlendioxides, vgl. auch DE-A 39 02 078).

**Tabelle 2**

| Ergebnisse der Phosphonium- bzw. Ammoniumfluorid-katalysierten HDI-Trimerisierung bei Verwendung von Wasser als Solvatisierungsmittel **S** für das Fluoridion | | | | |
|---|---|---|---|---|
| Versuch Nr. | Katalysatorkation | F⁻: H₂O im Katalysator ca. (molar) | Harztrübung [TE(F)] | Anteil Iminooxadiazindion im Trimerengemisch [mol-%] |
| 3-1a | Bu₄P⁺ | 1:1 | 0,7 | 39 |
| 3-1b | Bu₃(C₁₄H₂₉)P⁺ | 1:1 | 0,6 | 38 |
| 3-1c | Ph₃(Bu)P⁺ | 1:1 | 1,2 | 36 |
| 3-1d | R₃(Me)N⁺F⁻ | 1:1 | 1,4 | 36 |
| 3-2a | BU₄P⁺ | 1:1 | 0,5 | 42 |
| 3-2b | Bu₃(C₁₄H₂₉)P⁺ | 1:1 | 0,4 | 42 |
| 3-2c | Ph₃(Bu)P⁺ | 1:1 | 1,2 | 40 |
| 3-2d | R₃(Me)N⁺F⁻ | 1:1 | 1,2 | 39 |
| 3-3a | Bu₄P⁺ | 1:1 | 0,8 | 43 |
| 3-3b | Bu₃(C₁₄H₂₉)P⁺ | 1:1 | 0,3 | 45 |
| 3-3c | Ph₃(Bu)P⁺ | 1:1 | 1,3 | 43 |
| 3-3d | R₃(Me)N⁺F⁻ | 1:1 | 0,9 | 40 |
| 3-4 | Bu₄P⁺ | 1:5 | 0,5 | 35 |
| 3-5 | Bu₄P⁺ | 1:10 | 1,4 | 32 |

### Beispiel 4: Verwendung von Alkoholen als Solvatisierungsmittel S

Alle Beispiele, die sich auf die Herstellung von Produkten beziehen, die weniger als 30 mol-% Iminooxadiazindion im Trimerengemisch enthalten und/oder einen Trübungswert von 1,5 TE(F) überschreiten, sind Vergleichsbeispiele.

Stammlösung 1a wird als solche (Versuch 4-0), bzw. jeweils mit den in Tabelle 3 aufgeführten Alkoholen weiter auf die jeweils in Tabelle 3 aufgeführte Konzentration verdünnt, für HDI-Trimerisierungen in der unter 2a beschriebenen Weise verwendet (U_{NCO} jeweils ca. 20 %, Abstoppen durch Zugabe der dem F⁻-Verbrauch entsprechenden molaren Menge Dibutylphosphat, F⁻- Bedarf der Reaktion 20 -50 ppm F⁻, bezogen auf die Masse an eingesetztem HDI und die relative Masse des Fluoridions, 19 g/mol). Nur wenn der Katalysator in sehr hoher Konzentration eingesetzt wird (Versuch 4-0), sind gelegentlich geringe Feststoffbildungen in der Reaktionslösung zu beobachten. In diesen Fällen kann die Rohware vor der dünnschichtdestillativen Aufarbeitung entweder filtriert werden, was erheblich problemloser als bei den unter 2b und 2c aufgeführten Fällen gelingt, oder der Katalysator wird zur schnelleren homogenen Vermischung in das HDI eingedüst. Verfährt man so mit den unter 2b und 2c verwendeten Katalysatorlösungen, setzen sich die Düsen sofort mit Feststoff zu. Der Iminooxadiazindiongehalt hingegen liegt dann auf dem erfindungsgemäß hohen Niveau, wenn die Alkoholmenge (molar) ca. ein 20 faches der Fluoridionenkonzentration nicht wesentlich überschreitet, d.h., die Katalysatorkonzentration sollte, ca. 20 bis 30 % nicht unterschreiten (vgl. Tabelle 3).

**Tabelle 3**

| Ergebnisse der Phosphoniumfluorid-katalysierten HDI-Trimerisierung bei Verwendung monofunktioneller Alkohole als Solvatisierungsmittel **S** für das Fluoridion | | | | | |
|---|---|---|---|---|---|
| Versuch Nr. | Alkohol | Konzentration Bu₄P⁺ F⁻ ca. [%] | molares Verhältnis F⁻: ROH im Katalysator | Harztrübung [TE(F)] | Anteil Iminooxadiazindion im Trimerengemisch des Harzes [mol-%] |
| 4-0 | MeOH/ *iso*PrOH | 73 | 1: 2,4 | 0,5* | 45,2 |
| 4-1 | MeOH | 50 | 1 : 8,7 | 0,67 | 39,8 |
| 4-2 | MeOH | 40 | 1 : 13 | 0,54 | 40,2 |
| 4-3 | MeOH | 30 | 1 : 20 | 0,42 | 37,0 |
| 4-4 | MeOH | 5 | 1 : 165 | 0,39 | 15,8 |
| 4-5 | *iso*PrOH | 50 | 1 : 4,6 | 1,2 | 38,6 |
| 4-6 | *iso*PrOH | 5 | 1 : 88 | 0,94 | 23,0 |
| 4-7 | n-BuOH | 50 | 1 : 3,8 | 1,3 | 44,2 |
| 4-8 | n-BuOH | 5 | 1 : 71 | 0,69 | 23,1 |
| 4-9 | *iso*BuOH | 50 | 1 : 3,8 | 0,91 | 45,1 |
| 4-10 | *iso*BuOH | 5 | 1 : 71 | 0,52 | 22,8 |

| | | | | | |
|---|---|---|---|---|---|
| * bei Eindüsen des Katalysators, vgl. Text | | | | | |

### Beispiel 5: Verwendung von organischen Säuren als Solvatisierungsmittel S

In Stammlösung 1a wird jeweils äquimolar, bezogen auf die relative Masse des Fluoridions, 19 g/mol, die in Tabelle 4 aufgeführte organische Säure gelöst und die resultierenden Mischungen für HDI-Trimerisierungen in der unter 2a beschriebenen Weise verwendet (U_{NCO} jeweils ca. 20 %, Abstoppen durch Zugabe der dem F⁻-Verbrauch ensprechenden molaren Menge Dibutylphosphat, F⁻- Bedarf der Reaktion 20 - 50 ppm F⁻, bezogen auf die Masse an eingesetztem HDI und die relative Masse des Fluoridions, 19 g/mol). Es sind, unabhängig von der Art der Katalysatorzugabe, keine Feststoffbildungen während der Reaktion zu beobachten. Die Iminooxadiazindiongehalte sind Tabelle 4 zu entnehmen.

**Tabelle 4**

| Ergebnisse der Phosphoniumfluorid-katalysierten HDI-Trimerisierung bei Verwendung organischer Säuren als Solvatisierungsmittel **S** für das Fluoridion | | |
|---|---|---|
| Formel von **S** | pKₛ* (25°C, H₂O) | Iminooxadiazindionanteil in Trimerenmischung [mol-%] |
| HCOOH | 3,38-3,75 | ca. 35 |
| CH₃COOH | 4,75 | ca. 36 |
| HOCH₂(CH₃)₂CCOOH | 4,86-4,87 | ca. 40 |
| CH₃CH(OH)COOH | 4,12 | ca. 38 |
| (COOH)₂ | 1,27 | ca. 40 |
| HOOCCH₂COOH | 2,86 | ca. 45 |
| HOOC(CH₂)₂COOH | 4,21 | ca. 50 |
| HOOC(CH₂)₄COOH | 4,41-4,43 | ca. 35 |
| Phthalsäure | 2,58-2,89 | ca. 40 |
| Salicylsäure | 2,97-3,03 | ca. 35 |

| | | |
|---|---|---|
| *bei polybasigen Säuren ist immer der pK_{S1} angegeben; sind Bereiche angegeben, differieren die Angaben in der Literatur etwas (Handbook of Chemistry and Physics, 67th edition, 1986-1987CRC Press, Boca Raton, Florida, S. D-163 ff bzw. Beilstein, online Datenbank) | | |

### Beispiel 6: HDI/IPDI-Mischtrimerisierung

Eine Mischung aus 100 g (0,59 mol) HDI und 100 g (0,45 mol) Isophorondiisocyanat (IPDI) wird in einem 250 ml Vierhalskolben mit Innenthermometer, Rührer, Rückflußkühler, Gaseinleitungsrohr sowie Dosiereinrichtung für die Katalysatorlösung zunächst bei Zimmertemperatur und einem Druck von ca. 0,1 mbar im Verlauf einer Stunde von im Diisocyanatgemisch gelösten Gasen befreit und anschließend unter Durchleiten eines schwachen Stickstoffstromes auf 60°C Innentemperatur erhitzt. Anschließend werden bei dieser Temperatur im Verlaufe von ca. 20 Minuten portionsweise insgesamt 0,3 g (75 ppm F⁻) der Stammlösung 1a zugetropft und bei 60-70°C bis zu einem NCO-Gehalt der Mischung von 34,0 % trimerisiert, durch Zugabe von 0,2 g Di-n-butylphosphat abgestoppt, eine weitere Stunde bei 60°C nachgerührt und anschließend von nicht umgesetzten monomeren Diisocyanaten durch Dünnschichtdestillation in einem Kurzwegverdampfer bei 0,1 mbar und einer Temperatur des Heizmediums von 170°C abgetrennt. Das so erhaltene klare (Trübung = 1,1 TE(F)) und nahezu farblose Harz (65,6 g entsprechend 32,8 % Ausbeute) weist in reiner Form eine Viskosität von 23 000 mPa·s, einen NCO-Gehalt von 20,3 % und Restmonomergehalte von 0,07 % an HDI und 0,18 % an IPDI auf. Der Iminooxadiazindiongehalt in der Trimerenmischung beträgt 41,5 %.

### Beispiel 7:

100 g (0,51 mol) 1,3-Bis(isocyanatomethyl)cyclohexan (Fa. Aldrich) werden zunächst wie in Beispiel 6 beschrieben vorbehandelt und anschließend durch portionsweise Zugabe der Polyfluorid-Stammlösung 1a, summarischer Katalysatorbedarf: 42 ppm F⁻ bei 58-60°C, innerhalb von 3 Stunden auf einen NCO-Gehalt von 36,6 % trimerisiert. Anschließend wird durch Zugabe von 100 mg Di-n-octylphosphat abgestoppt, eine weitere Stunde bei 60°C nachgerührt und von nicht umgesetztem 1,3-Bis(isocyanatomethyl)cyclohexan durch Dünnschichtdestillation in einem Kurzwegverdampfer bei 0,2 mbar und einer Temperatur des Heizmediums von 140°C abgetrennt. Das so erhaltene, klare und nahezu farblose Harz (33,5 g entsprechend 33,5 % Ausbeute) weist einen NCO-Gehalt von 19,9 % auf und ist in reiner Form bei Zimmertemperatur (20-25°C) gerade noch fließfähig. Die Viskosität der 80 %igen Lösung in n-Butylacetat beträgt 1530 mPa·s bei einem NCO-Gehalt von 15,9 %. Der Restmonomergehalt beläuft sich auf 0,07 % an 1,3-Bis(isocyanatomethyl)cyclohexan. Der Iminooxadiazindionanteil im Trimerengemisch beläuft sich auf 45,2 %.

## Patentansprüche

1. Verfahren zur Herstellung von Polyisocyanaten mit mindestens 30 mol-% an Iminooxadiazindiongruppen (asymmetrische Trimere), bezogen auf die Gesamtmenge an Isocyanurat- und Iminooxadiazindiongruppen, durch katalytisch induzierte Trimerisierung organischer Di- oder Polyisocyanate mit einem (mittleren) Molekulargewicht von 140 - 600 g/mol mit unabhängig voneinander aliphatisch, cycloaliphatisch und/oder araliphatisch gebundenen Isocyanatgruppen, **dadurch gekennzeichnet, daß** als Trimerisierungskatalysator quaternäre Ammonium- bzw. Phosphoniumfluoride der Formel
R₄E⁺ F⁻ (I),
wobei
E für N oder P steht und
R für gleiche oder verschiedene, gegebenenfalls verzweigte, aliphatische, aromatische und/oder araliphatische C₁-C₂₀ Reste steht, wobei zwei oder mehrere Substituenten R untereinander und mit dem Stickstoff-bzw. Phosphoratom auch gesättigte oder ungesättigte Cyclen bilden können,
in Abmischung mit Solvatisierungsmitteln **S** für das Fluoridanion eingesetzt werden, wobei **S** eine reine Verbindung oder ein Gemisch verschiedener Stoffe aus der Gruppe der protischen Verbindungen mit einem pKₛ-Wert größer als 2 (bestimmt in H₂O bei 25°C) sowie Oxalsäure, jedoch kein höherfunktioneller Alkohol (Di- bzw. Polyol) sowie HF, sein kann, unter der Voraussetzung, daß das molare Verhältnis von **S** zu Fluoridion (F⁻) den Wert 10 nicht überschreitet.

2. Verfahren nach Anspruch 1, **dadurch gekennzeichnet, daß** als zu trimerisierende Isocyanatkomponente aliphatische Diisocyanate eines Molekulargewicht(sbereich)es von 140 - 300 g/mol als reine Verbindungen oder in beliebiger Abmischung untereinander eingesetzt werden.

3. Verfahren nach Anspruch 1, **dadurch gekennzeichnet, daß** als Trimerisierungskatalysator quaternäre Ammonium- bzw. Phosphoniumfluoride der Formel (I) in Abmischung mit monofunktionellen Alkohol(gemische)en eines (mittleren) Molekulargewichtsbereiches von 32 - 250 g/mol eingesetzt werden, wobei die Konzentration des quaternären Ammonium- bzw. Phosphoniumfluorides 20 Masse-% in der Mischung nicht unterschreitet.

4. Verfahren nach Anspruch 1, **dadurch gekennzeichnet, daß** als Trimerisierungskatalysator quaternäre Ammonium- bzw. Phosphoniumfluoride der Formel (I) in Abmischung mit organischen Säuren eines pKₛ-Wertes größer als 2 (in H₂O bei 25°C) sowie Oxalsäure eingesetzt werden, wobei das molare Verhältnis von organischer Säure zu Fluoridion, F⁻, den Wert 10 nicht überschreitet.

5. Verfahren nach Anspruch 1, **dadurch gekennzeichnet, daß** als Trimerisierungskatalysator quaternäre Ammonium- bzw. Phosphoniumfluoride der Formel (I) in Abmischung mit Wasser eingesetzt werden, wobei das molare Verhältnis von Wasser zu Fluoridion, F⁻, den Wert 10 nicht überschreitet.

## Claims

1. A process for the production of polyisocyanates which contain at least 30 mol% of iminooxadiazinedione groups (asymmetric trimers), relative to the total quantity of isocyanurate and iminooxadiazinedione groups, by catalytically induced trimerisation of organic di- or polyisocyanates having a(n average) molecular weight of 140-600 g/mol with mutually independently aliphatically, cycloaliphatically and/or araliphatically bound isocyanate groups, **characterised in that** the trimerisation catalyst used comprises quaternary ammonium and phosphonium fluorides of the formula
R₄E⁺F⁻ (I),
wherein
E denotes N or P and
R denotes identical or different, optionally branched, aliphatic, aromatic and/or araliphatic C₁-C₂₀ residues, wherein two or more substituents R may together also form saturated or unsaturated cycles with the nitrogen or phosphorus atom,
blended with solvating agents **S** for the fluoride anion, wherein **S** may be a pure compound of a mixture of various substances from the group of protic compounds having a pKₐ value of greater than 2 (determined in H₂O at 25°C) and oxalic acid, but not a more highly functional alcohol (di- or polyol) and HF, provided that the molar ratio of **S** to fluoride ion (F⁻) does not exceed a value of 10.

2. A process according to claim 1, **characterised in that** the isocyanate component to be trimerised comprises aliphatic diisocyanates of a molecular weight (range) from 140-300 g/mol as pure compounds or in any desired blend with each other.

3. A process according to claim 1, **characterised in that** the trimerisation catalyst used comprises quaternary ammonium or phosphonium compounds of the formula (I) blended with monofunctional alcohols/alcohol mixtures of a(n average) molecular weight range from 32-250 g/mol, wherein the concentration of the quaternary ammonium or phosphonium fluoride in the mixture is no lower than 20 wt.%.

4. A process according to claim 1, **characterised in that** the trimerisation catalyst used comprises quaternary ammonium or phosphonium fluorides of the formula (I) blended with organic acids of a pKₐ value of greater than 2 (in H₂O at 25°C) and oxalic acid, wherein the molar ratio of organic acid to fluoride ions, F⁻, does not exceed a value of 10.

5. A process according to claim 1, **characterised in that** the trimerisation catalyst used comprises quaternary ammonium or phosphonium fluorides of the formula (I) blended with water, wherein the molar ratio of water to fluoride ions, F⁻, does not exceed a value of 10.

## Revendications

1. Procédé de préparation de polyisocyanates contenant au moins 30 % en mol de groupes iminooxadiazinedione (trimères asymétriques) par rapport à la quantité totale de groupes isocyanurate et iminooxadiazinedione, par trimérisation induite par un catalyseur de di- ou polyisocyanates organiques ayant une masse molaire (moyenne) de 140 à 600 g/mol et contenant des groupes isocyanate liés indépendamment les uns des autres par des liaisons aliphatiques, cycloaliphatiques et/ou araliphatiques, **caractérisé en ce que** l'on utilise comme catalyseur de la trimérisation des fluorures d'ammonium et de phosphonium quaternaires de formule
R₄E⁺ F⁻ (I)
dans laquelle
E représente N ou P, et
les R représentent des restes aliphatiques, aromatiques et/ou araliphatiques en C₁-C₂₀ identiques ou différents, éventuellement ramifiés, et deux ou plusieurs substituants R peuvent aussi éventuellement former entre eux et avec l'atome d'azote ou de phosphore des cycles saturés ou insaturés,
en mélange avec des agents de solvatation S pour l'anion fluorure, S pouvant être un composé pur ou un mélange de différentes substances du groupe des composés protiques ayant un pKₛ supérieur à 2 (déterminé dans H₂O à 25°C), ainsi que l'acide oxalique, mais non un alcool polyfonctionnel (di- ou polyol) ni HF, à condition que le rapport molaire de S à l'ion fluorure, F⁻, ne dépasse pas la valeur de 10.

2. Procédé selon la revendication 1, **caractérisé en ce que** l'on utilise comme constituant isocyanate à trimériser des diisocyanates aliphatiques ayant une masse molaire ou un domaine de masse molaire de 140 à 300 g/mol sous forme de composés purs ou sous forme de mélanges quelconques entre eux.

3. Procédé selon la revendication 1, **caractérisé en ce que** l'on utilise comme catalyseur de trimérisation des fluorures d'ammonium ou de phosphonium quaternaire de formule (I) en mélange avec des alcools ou des mélanges d'alcools monofonctionnels ayant un domaine de masse molaire (moyenne) de 32 à 250 g/mol, la concentration du fluorure d'ammonium ou de phosphonium quaternaire ne dépassant pas 20 % en masse du mélange.

4. Procédé selon la revendication 1, **caractérisé en ce que** l'on utilise comme catalyseur de trimérisation des fluorures d'ammonium ou de phosphonium quaternaire de formule (I) en mélange avec des acides organiques ayant un pKₛ supérieur à 2 (dans H₂O à 25°C), ainsi qu'avec l'acide oxalique, le rapport molaire de l'acide organique à l'ion fluorure F⁻ ne dépassant pas la valeur 10.

5. Procédé selon la revendication 1, **caractérisé en ce que** l'on utilise comme catalyseur de trimérisation des fluorures d'ammonium ou de phosphonium quaternaire de formule (I) en mélange avec de l'eau, le rapport molaire de l'eau à l'ion fluorure F⁻ ne dépassant pas la valeur 10.
